**Europäisches Patentamt**

(19) **European Patent Office**

**Office européen des brevets**

(11) Publication number: **0 083 372**
**B1**

# (12) EUROPEAN PATENT SPECIFICATION

(45) Date of publication of patent specification: **18.05.88**

(21) Application number: **82902507.1**

(22) Date of filing: **08.07.82**

(86) International application number:
**PCT/US82/00929**

(87) International publication number:
**WO 83/00284 03.02.83 Gazette 83/04**

(51) Int. Cl.⁴: **A 61 K 9/52**

(54) **SUSTAINED RELEASE THEOPHYLINE.**

(30) Priority: **15.07.81 US 283446**
**27.04.82 US 372300**

(43) Date of publication of application:
**13.07.83 Bulletin 83/28**

(45) Publication of the grant of the patent:
**18.05.88 Bulletin 88/20**

(84) Designated Contracting States:
**AT BE CH DE FR GB LI LU NL SE**

(56) References cited:
**US-A-2 811 483**
**US-A-2 853 420**
**US-A-2 928 770**
**US-A-3 081 233**
**US-A-3 109 775**
**US-A-3 247 066**
**US-A-3 344 029**
**US-A-3 400 185**

The file contains technical information
submitted after the application was filed and
not included in this specification

(73) Proprietor: **Key Pharmaceuticals, Inc.**
**2000 Galloping Hills Road**
**Kenilworth New Jersey 07033 (US)**

(72) Inventor: **HSIAO, Chiin Hsiung**
**Key Pharmaceuticals, Inc. 18425 N.W. 2nd**
**Avenue**
**Miami, FL 33169 (US)**

(74) Representative: **Werner, Hans-Karsten, Dr. et al**
**Deichmannhaus am Hauptbahnhof**
**D-5000 Köln 1 (DE)**

(56) References cited:
**US-A-3 835 221**
**US-A-3 976 764**
**US-A-4 083 949**
**US-A-4 173 626**

**HAGERS HANDBUCH DER
PHARMAZEUTISCHEN PRAXIS, vol. VII, 4th
edition, part B, 1977, Springer-Verlag, Berlin,
DE;**

Courier Press, Leamington Spa, England.

## Description

A zero order release theophylline product, THEO-DUR®, has received widespread acceptance in the marketplace and among the medical profession as a broncho-dilatator. It is desired to provide a sustained release broncho-dilatator which is in a form more suitable for administration to children and the elderly.

U.S. Patent 4,083,949 discloses an oral form of medication and a method for producing it. The oral form disclosed releases the active ingredient in the gastrointestinal tract at a constant rate. The dosage form may be comprised of spheroidal shaped particles of active ingredient which are coated with materials such as ethyl cellulose in order to provided sustained release of the active ingredient.

U.S. Patent 3,976,764 discloses a solid therapeutic preparation for internal use remaining in stomach for a long period of time and gradually releasing an active ingredient contained therein into gastric juice while remaining, said preparation having been prepared by impregnation of the active ingredient into a body of empty globular shell or a granular lump in small size of a material having high buoyancy.

There are disclosed hollow capsules which are coated with a first undercoating and then with a further coating which includes active ingredient.

U.S. Patent 3,400,185 discloses a process for forming spherical pellets of pharmaceutically active materials. More particularly, it relates to a process of forming spherical pellets from pharmaceutically active material which are suitable for coating a variety of coating materials including enteric coating materials.

Although the dosage forms described above may offer advantages with respect to delivery the active ingredient to the patient, each of the disclosures does not take into consideration all of the factors which must be considered when attempting to provide the correct therapeutic blood level of theophylline to a young or elderly patient via an oral formulation. Accordingly, the above described formulations suffer from one or more disadvantages thus indicating a clear need for an oral dosage formulation such as that being presented herein by the present invention.

Hagers Handbuch der Pharmazeutischen Praxis 7. Bd. Teil B, S. 141, Springer 1977 just describes the usage of ethyl cellulose and hydroxypropyl cellulose as coating for drugs.

In accordance with the present invention there are provided micropellets of sustained release theophylline which are designed for administration to children or the elderly who may have difficulty swallowing a relatively large tablet. The micropellets are provided in a capsule as container of the dosage unit, each capsule containing about 1000 microparticles or less to yield the total desired dosage. For example, in one embodiment, a 100 mg dosage of theophylline is provided with 845 microparticles.

The microparticles are provided by coating theophylline in micronized form onto a seed preferably a sugar seed having 0.18—0.25 mm (60—80 mesh) in diameter with polyvinylpyrrolidone having a molecular weight of from 30,000 to 50,000, with a molecular weight of about 40,000 being a preferred embodiment ("Kollidon® 30"). A sustained release coating is provided of cellulose derivatives. In a preferred embodiment a mixture of ethylcellulose (Dow, "Ethocel® N-10") and hydroxypropylcellulose (Hercules, "Klucel® LF" is used. The average diameter of each particle is roughly about 0.6 mm.

More precisely the present invention provides a sustained release dosage formulation of theophylline to be sprinkled into food consisting of a plurality of micropellets, the sum of such micropellets forming a dosage unit of theophylline, each of said micropellets being based upon a seed being coated by a first coating mixture of theophylline and polyvinylpyrrolidone, and being further coated by a second coating mixture of ethylcellulose and hydroxypropylcellulose, said micropellets being included in a container in form of a capsule.

The following example illustrates the invention:

Example

3.2 kg polyvinylpyrrolidone (mw 40,000, Kollidon® 30) is dissolved in 32 l isopropanol. Therein there is dispersed 12.8 kg of micronized theophylline. Into a Glatt column (15.2 cm (6 inch) Glatt CPCG) there is charged 4.0 kg sugar, 0.18—0.25 mm in diameter.

After the air suspension system is in operation with the sugar, the dispersed theophylline is charged into the Glatt column with the inlet air having a temperature of 60°C and the spray pressure at 4 bars, and the spray rate being 100 ml/min.

After completion of the above procedure, the Glatt column is stopped, and the product reserved as "Theophylline pellets, Active I".

A second batch of 3.2 kg polyvinylpyrrolidone (mw 40,000, Kollidon® 30) is dissolved into 32.0 l isopropanol, and dispersed into the resultant mixture is 12.8 kg micronized theophylline. 4.0 kg of Theophylline pellets, Active I are then charged into the same Glatt column under the same conditions of temperature, pressure and rate. The second batch having the theophylline dispersed therein is then charged into the Glatt column to further build up the coating.

The Glatt column is emptied and the product labelled "Theophylline pellets Active II".

A mixture of 13.2 l chloroform and 3.3 l methanol is prepared, into which are dispersed 992.0 g ethylcellulose (Ethocel N-10, Dow) and 329.0 g hydroxypropyl cellulose Hercules, Klucel® LF). Into the Glatt column is charged 19.0 kg of Theophylline pellets, Active II, which are then coated with the coating mixture under conditions of 30°C, spray pressure 3 bars and spray rate 100 ml/min. The resultant coated pellets are small white micropellets. To make up a dosage unit of 100 mg, 845 of such micropellets are placed into capsules for convenience. For a child or an elderly patient, the microparticles are typically sprinkled into food which is then taken as part of a meal, to provide the sustained theophylline effect.

**Claims**

1. A sustained release dosage formulation of theophylline to be sprinkled into food consisting of a plurality of micropellets, the sum of such micropellets forming a dosage unit of theophylline, each of said micropellets being based upon a seed being coated by a first coating mixture of theophylline and polyvinylpyrrolidone, and being further coated by a second coating mixture of ethylcellulose and hydroxypropylcellulose, said micropellets being included in a container in form of a capsule.

2. The dosage formulation of Claim 1 further characterized in that said polyvinylpyrrolidone has a molecular weight of 30,000 to 50,000.

3. The dosage formulation of Claim 2 further characterized in that said polyvinylpyrrolidone has a molecular weight of about 40,000.

4. The dosage formulation of Claim 1 further characterized in that said seed is sugar having a size of 0.18—0.25 mm (60—80 mesh).

5. The dosage formulation of Claim 1 further characterized in that said micropellets have diameters of about 0.6 mm.

**Patentansprüche**

1. Theophyllin-Dosierungsformulierung mit verlängerter Wirkstoff-Abgabe, die sich in eine Speise streuen läßt, bestehend aus einer Vielzahl von Mikropellets, wobei die Summe solcher Mikropellets eine Dosierungseinheit Theophyllin bildet, wobei jedes der Mikropellets auf einem Keimling aufbaut, auf dem eine erste Beschichtungsmischung aus Theophyllin und Polyvinylpyrrolidon aufgetragen ist und weiterhin eine zweite Beschichtungsmischung aus Ethylcellulose und Hydroxypropylcellulose aufgetragen ist, und wobei die Mikropellets in einem Behälter in Form einer Kapsel enthalten sind.

2. Dosierungsformulierung nach Anspruch 1, weiterhin dadurch gekennzeichnet, daß das Polyvinylpyrrolidon ein Molekulargewicht von 30 000 bis 50 000 hat.

3. Dosierungsformulierung nach Anspruch 2, weiterhin dadurch gekennzeichnet, daß das Polyvinylpyrrolidon ein Molekulargewicht von 40 000 hat.

4. Dosierungsformulierung nach Anspruch 1, weiterhin dadurch gekennzeichnet, daß der Keimling Zucker mit einer Größe von 0,18 bis 0,25 mm (60—80 mesh) ist.

5. Dosierungsformulierung nach Anspruch 1, weiterhin dadurch gekennzeichnet, daß die Mikropellets Durchmesser von etwa 0,6 mm haben.

**Revendications**

1. Formule de dosage de théophylline à liberation prolongée, à répandre dans des aliments, qui comprend une pluralité de micro-boulettes, la somme des micro-boulettes formant une unité de dosage de théophylline, chacune desdites micro-boulettes étant basée sur une semence étant enduite par un premier mélange de revêtement de théophylline et polyvinylpyrrolidone, et étant de plus enduite par un second mélange de revêtement d'éthylcellulose et hydroxycellulose, lesdites micro-boulettes étant incorporées dans un récipient en forme d'une capsule.

2. La formule de dosage de la revendication 1, caractérisée de plus en ce que ladite polyvinylpyrrolidone a un poids moléculaire de 30 000 à 50 000.

3. La formule de dosage de la revendication 2, caractérisée de plus en ce que ladite polyvinylpyrrolidone a un poids moléculaire d'environ 40 000.

4. La formule de dosage de la revendication 1, caractérisée de plus en ce que ladite semence est du sucre ayant une taille de 0,18—0,25 mm (maille 60—80).

5. La formule de dosage de la revendication 1, caractérisée de plus en ce que lesdites micro-boulettes ont des diamètres d'environ 0,6 mm.